Europäisches Patentamt

European Patent Office

Office européen des brevets

(11) Publication number:

**0 067 293**
**B1**

(12) **EUROPEAN PATENT SPECIFICATION**

(45) Date of publication of patent specification: **25.06.86**

(21) Application number: **82103473.3**

(22) Date of filing: **24.04.82**

(51) Int. Cl.⁴: **A 61 K 37/64**

(54) Method for isolating alpha-1-antitrypsin.

(30) Priority: **01.05.81 US 259644**
**01.03.82 US 353708**

(43) Date of publication of application:
**22.12.82 Bulletin 82/51**

(45) Publication of the grant of the patent:
**25.06.86 Bulletin 86/26**

(84) Designated Contracting States:
**DE FR GB SE**

(56) References cited:
**GB-A-1 034 186**
**GB-A-1 034 187**
**GB-A-1 062 434**

**CHEMICAL ABSTRACTS, vol. 83, 1975, page
178, no. 174143c, Columbus, Ohio, USA, T.E.
CREIGHTON: "Interactions between cysteine
residues as probes of protein conformation.
Disulfide bond between Cys-14 and Cys-38 of
the pancreatic trypsin inhibitor"**

The file contains technical information
submitted after the application was filed and
not included in this specification

(73) Proprietor: **Medical Research Institute of San
Francisco
Clay and Webster Streets
San Francisco California (US)**

(72) Inventor: **Glaser, Charles Barry
284 Wawona Street
San Francisco California (US)**

(74) Representative: **Reitzner, Bruno, Dr. et al
Patentanwälte Dipl.-Ing. R. Splanemann Dr. B.
Reitzner Tal 13
D-8000 München 2 (DE)**

(56) References cited:

**BIOLOGICAL ABSTRACTS, vol. 69, 1980,
abstract no. 44137, J.A. PIERCE et al.:
"Improved identification of antitrypsin
phenotypes through isoelectric focusing with
dithioerythritol"**

Courier Press, Leamington Spa, England.

## 0 067 293

**Description**

This invention relates to a method for isolating alpha-1-antitrypsin from a mixture of blood plasma proteins, preferably Cohn fraction IV-I, a subfraction obtained from pooled human plasma, in solution.

Alpha-1-antitrypsin is a major inhibitor of proteolytic enzymes in human serum. There are about 70,000 to 80,000 people who have a genetic deficiency which results in serum levels of alpha-1-antitrypsin that are only 10—15% of normal. These individuals are strongly disposed to pulmonary emphysema. The lung damage results from the digestive action of improperly regulated enzymes due to the alpha-1-antitrypsin deficiency. There are a number of other acute clinical conditions, including sepsis, pancreatitis, respiratory failure, and cardiopulmonary bypass, where there are indications that excessive proteolysis may be an important contributing factor.

The present invention provides a process for obtaining purified alpha-1-antitrypsin for treatment of these disease states. The process produces the desired product through a sequence of steps which are collectively inexpensive, simple, reproducible, and which create a relatively pure product in high yield that is safe for therapeutic use and applicable to large scale industrial processing.

"Isolation and Characterization of Alpha-1-Antitrypsin from the Cohn Fraction IV-I of Human Plasma", by Charles B. Glaser, Lucy Karic and Robert Fallat, *Preparative Biochemistry*, 5(4), 1975 pages 333—348, describes the isolation of alpha-1-antitrypsin from pooled human plasma utilizing ion exchange, biospecific affinity and gel exclusion chromatographic procedures.

"Evaluation of Alpha-1-Antitrypsin as a Potential Therapeutic Agent", by Allen B. Cohen and Harold James, DHEW Publication #(NIH)78-1422, pages 326—388, describes a method for isolating alpha-1-antitrypsin and its potential therapeutic utility.

This method includes the following sequence of steps:

a) Ammonium Sulfate Precipitation;

b) Cibracon Blue®-Sepharose® chromatography;

c) QAE Sephadex® chromatography, (first at pH 8.6 and then at pH 6.5);

d) Thiol Sepharose® 4B chromatography.

Purification of alpha-1-antitrypsin by chromatographic techniques utilizing the disulfide group in alpha-1-antritrypsin is disclosed in "Thiol-Disulfide Interchange Chromatography Using Sepharose®-Linked Thiol Compounds to Separate Plasma Proteins", by C. B. Laurell, Eva Thulin and R. P. Bywater, *Analytical Biochemistry*, 81 (1977), pages 336—345, and in "Purification of Alpha-1-Antitrypsin from Plasma through Thiol-Disulfide Interchange", by C. B. Laurell, J. Pierce, U. Persson and F. Thulin, *Eur. J. Biochem.*, 57 (1975) pages 107—113.

"Improved Identification of Antritypsin Phenotypes Through Isoelectric Focusing with Dithioerythritol", by John A. Pierce and Bibiana G. Eradio, *J. Lab. Clin. Med.*, December 1979, pages 826—831, describes the use of dithiothreitol and dithioerythritol for identifying antitrypsin phenotypes through isoelectric focusing.

Summary of the invention

In accordance with the present invention there is provided a method for isolating alpha-1-antitrypsin from a mixture of blood plasma proteins in solution comprising: (a) breaking sufficient disulfide bonds in said proteins to destabilize them, (b) precipitating the destabilized proteins, (c) and recovering non-precipitated alpha-1-antitrypsin from said solution.

The term "destabilize" is used in a relative sense and refers to the solubility of the proteins in the solution being treated. A destabilized protein is rendered less soluble than the protein being isolated (alpha-1-antitrypsin in the preferred embodiment) and can therefore be selectively precipitated by appropriate adjustment of the solution conditions, as will be discussed.

It will be appreciated that all disulfide bonds present need not be broken to obtain this relative instability. The process contemplates breaking a sufficient number of the bonds so that the desired selective precipitation is obtained.

In the preferred embodiment the precipitated proteins are separated first before recovering alpha-1-antitrypsin from the supernatant solution. The disulfide bonds are preferably broken with an effective concentration of any suitable reducing agent that is strong enough to reduce protein disulfide bonds, but not reduce other protein bonds such as peptide linkages or other groups which would cause a fragmentation of the protein. Typical reducing agents which might be utilized are monothiols such as 2-percaptoethanol, mercaptoacetic acid, and cysteine. Suitable nonthiol reducing agents are exemplified by sodium borohydride. Preferably the reducing agent is a dithiol such as dithiothreitol or its steroisomer dithioerythritol.

The dithiols are more effective in this process, forming a ring structure upon reducing a disulfide group to sulfhydryls. This ring formation drives the reaction to completion via favorable entropic effects. Accordingly, sterically favorable dithiols can be used at a concentration of 0.01M or higher, usually 0.03—0.1M. The less effective reducing agents such as monothiols require a minimum concentration of 0.1M, usually 0.5M—1.5M.

The protein solution is preferably adjusted with a buffer to a pH of about 6.0—8.5. Suitable buffers include phosphate and tris. At higher pHs the protein becomes less stable but the higher pHs could be

2

utilized if desired. At pHs below about 6 the alpha-1-antitrypsin tends to denature and aggregate and therefore such lower pHs would usually be less satisfactory.

In the protein precipitation step any agent that may lower the solubility of proteins may be utilized such as polyethylene glycol, alcohols such as ethyl alcohol, guanidine, and urea. In the preferred embodiment the protein is salted out with the addition of sufficient salts which may be those typically used for precipitating proteins and preferably will be a divalent salt such as a sulfate. the precipitated proteins may then be separated from the supernatant by any suitable technique such as centrifuging.

Alpha-1-antitrypsin is recovered from the supernatant by any one of several methods. Ultrafiltration with hollow fibers may provide acceptable results in many cases. Best results have thus far been obtained by chromatographic separation particularly with DEAE-cellulose columns, although other ion exchange resins or other chromatographic means may be used (gel filtration, hydroxyapatite, immuno-adsorbant, affinity columns containing Cibacron® blue, concanavalin A, DNA, etc.). By selecting a chromatographic column and solvent conditions including pH and ion concentration under which alpha-1-antitrypsin binds more strongly than the other proteins, protein concentrations in excess of the binding capacity of the column can be introduced. The alpha-1-antitrypsin will bind to the column while the more weakly binding proteins pass through. The alpha-1-antitrypsin can then be eluted with an appropriate stronger solvent. This technique permits the loading of larger amounts of protein onto the column that would otherwise be possible.

The present method is particularly effective in that it utilizes the disulfide bond which is present in most known plasma proteins. The reducing agent such as dithiothreitol effectively reduces many of these protein disulfide bonds even in the absence of other denaturants. A primary function of the disulfide bonds is to stabilize the protein structure. Consequently when disulfide bonds are broken the proteins become destabilized in solution. The destabilized proteins may then be readily precipitated by suitable techniques such as salting, heating, change in pH, addition of solvents and the like.

The process is presently believed to be effective because alpha-1-antitrypsin has only a single disulfide bond and this bond is not involved in stabilizing the protein chain. The alpha-1-antitrypsin is not thus destabilized with the reduction step and remains in the supernatant solution. If desired, this single disulfide bond can be reconstituted after the reduction step.

A potential collateral benefit is obtained from use of the present method. The reducing conditions that may be used also serve to effectively destroy the infective properties of any hepatitis virus in the protein source. It is thought that, because the viral coat contains various disulfide containing proteins, it will be denatured under the process conditions and the virus will thereby lose its infectivity.

In the preferred embodiment human blood plasma is utilized as the protein solution. Preferably the present process utilizes the Cohn IV-I fraction of blood plasma as a starting material because it is enriched in alpha-1-antitrypsin (6—10% of the protein) and is currently a relatively unused side fraction during the production of albumin and gamma globulin.

Cohn IV-I paste contains approximately 25—35% protein, 30—35% alcohol, 30—40% $H_2O$ and consists of at least the following proteins: albumin (~15—20%), alpha-1-antitrypsin (~6—10%), alpha-1-antichymotrypsin (~4—6%), ceruloplasmin (2.5%), haptoglobulin (~2%), antithrombin III (~1%), transferrin (~7%), hemopexin, alpha-2-plasmin inhibitor, alpha-1-lipoprotein, plasminogen, fibrinogen, $C_2$, $C_3$, alpha-1-acid glycoprotein, $C_1$ esterase inhibitor, inter-alpha-trypsin inhibitor, $C_1$ esterase inactivator.

Regardless of the source, it is preferable that the protein solution used in the process be diluted so that it contains less than about 50 mg/ml of protein. Where the concentration is appreciably higher, excessive alpha-1-antitrypsin may be lost through occlusion in the precipitated proteins.

Experimental
Materials and methods
Cohn Fraction IV-I was obtained as a frozen, homogenized paste from Cutter Laboratories, Berkeley, California. The trypsin inhibitory capacity (TIC) was determined against crystallized, salt-free, bovine trypsin (Worthington Chemical Company, Freehold, New Jersey) using benzoyl - DL - arginine - p - nitroanilide as a substrate. The percentage of active trypsin was determined by titration with p-nitrophenyl p'-guanidinobenzoate. The concentration of alpha-1-antitrypsin was determined by the radial immuno-diffusion method (RID) using rabbit anti-serum to human alpha-1-antitrypsin (Kallestad Labs, Chaska, Minnesota). Specific activity was estimated by dividing the TIC in mg/ml by the protein concentration as determined by the method of Lowry, et al., or by the weight of lyophilized protein (with a 15% moisture correction). The extinction coefficient of alpha-1-antitrypsin is

$$E_{1\,cm,\,280\,nm}^{0.1\%}=0.50$$

and was used as a measure of protein concentration and specific activity for purified products.

The final product was phenotyped by starch gel electrophoresis with an acidic, discontinuous buffer system, followed by crossed antigen-antibody electrophoresis.

Analytical SDS acrylamide gel electrophoresis was performed in 9% gels by the method of Laemmli (1970). Immunoelectrophoresis was carried out by standard procedures on an LKB system.

Cohn fraction IV-I paste may be activated by solubilizing in 0.1M buffer at pH 8.5 (phosphate, Tris) and

dialyzing against 0.01M buffer, pH 7.6 which serves the purpose of removing the alcohol. As will be shown below in Example 1, dialysis to remove alcohol is not essential.

Throughout the examples the analytical methods referred to are as described in the following references:

TIC=B. F. Erlanger, N. Kokowsky and W. Cohen, *Arch. Biochem. Biophys.*, 95, 271—278 (1961).

RID=M. Mancini, A. P. Carbonara and J. F. Heremans, *Immunochemistry*, 29, 235—254 (1965).

Lowry=O. H. Lowry, N. L. Rosebrough, A. J. Farr and H. J. Randall, *J. Biol. Chem.*, 193, 265—272 (1951).

Example 1

Activation and salting out of alpha-1-antitrypsin

100 g of Cohn IV-I was dissolved in 1.0 l of 0.1M phosphate at pH 8.5 and the solution was stirred for ~3 hours. Undissolved material was removed by filtration. The solution was divided into four aliquots as follows: One aliquot (A) was concentrated directly to ~20 mg/ml. A second aliquot (B) was first dialysed against 0.01M phosphate, pH 8.5 using a DC-2 Amicon hollow fiber with a 10,000 M.W. cutoff, and then concentrated to ~20 mg/ml. A third aliquot (C) was concentrated by ultrafiltration to ~50 mg/ml. The fourth aliquot (D) was dialyzed against 0.01M phosphate at pH 8.5 and then concentrated to ~50 mg/ml. Four 10 ml aliquots of each of these alpha-1-antitrypsin solutions (A—D) were prepared. Half of the solutions were treated with 0.1M dithiothreitol (DTT) for either 3 or 24 hours at 25°C, and all samples were then salted out to a final concentration of 50% $(NH_4)_2SO_4$ at 5°C and stirred overnight. This was followed by centrifugation at 3000 rpm for 2 hours. The precipitate was washed with 50% $(NH_4)_2SO_4$ and the supernatants combined. The initial and final trypsin inhibitory capacity were determined for each sample by the TIC assay, as well as the protein content by the method of Lowry. The results are summarized in Table I.

4

TABLE I

| | DTT reduction (0.1M) | Reduction time (h) | Initial protein (mg) | Final protein (mg) | Protein recovery (%) | Initial | Final | Alpha-1-anti-trypsin recovery (by TIC) (%) | Specific TIC mg trypsin inhibited mg protein initial | Final | Purification factor |
| | | | | | | Alpha-1-antitrypsin (by TIC) (mg) corrected** | | | | | |
|---|---|---|---|---|---|---|---|---|---|---|---|
| A) 20.2 mg.ml no dialysis | + | 3 | 202.0 | 14.0* | * | 12.1 | 6.0* | * | .06 | .43 | 7.2 |
| | + | 24 | 202.0 | 20.0 | 10.0 | 12.1 | 9.1 | 75 | .06 | .46 | 7.7 |
| | − | 3 | 202.0 | 70.0 | 35.0 | 12.1 | 9.0 | 74 | .06 | .11 | 2.2 |
| | − | 24 | 202.0 | 77.0 | 38.0 | 12.1 | 9.1 | 75 | .06 | .12 | 2.0 |
| B) 21.2 mg/ml after dialysis | + | 3 | 212.0 | 28.0 | 13.2 | 12.0 | 10.9 | 91 | .057 | .39 | 6.8 |
| | + | 24 | 212.0 | 29.2 | 13.8 | 12.0 | 10.0 | 84 | .057 | .34 | 6.0 |
| | − | 3 | 212.0 | 72.8 | 34.3 | 12.0 | 11.5 | 96 | .057 | .16 | 2.8 |
| | − | 24 | 212.0 | 91.0 | 43.0 | 12.0 | 9.4 | 79 | .057 | .10 | 1.8 |
| C) 48.7 mg/and no dialysis | + | 3 | 487 | 57.6 | 11.8 | 24.1 | 20.8 | 86 | .050 | .36 | 7.2 |
| | + | 24 | 487 | 48.0 | 9.9 | 24.1 | 18.4 | 77 | .050 | .38 | 7.6 |
| | − | 3 | 487 | 154. | 32.0 | 24.1 | 19.3 | 80 | .050 | .13 | 2.6 |
| | − | 24 | 487 | 72.6 | 15.0 | 24.1 | 18.7 | 78 | .050 | .26 | 5.2 |
| D) 49.5 mg/ml after dialysis | + | 3 | 497 | 70.5 | 14.2 | 24.6 | 19.0 | 77 | .050 | .27 | 5.4 |
| | + | 24 | 495 | 46.0 | 9.3 | 24.6 | 16.2 | 66 | .050 | .35 | 7.0 |
| | − | 3 | 495 | 158.0 | 32.0 | 24.6 | 16.8 | 68 | .050 | .11 | 2.2 |
| | − | 24 | 495 | 144.0 | 29.1 | 24.6 | 17.2 | 70 | .050 | .12 | 2.4 |

*Lost some of protein prior to dialysis.
**Corrected for trypsin activity (69%) and for factor 2.17 (obtained by using M.W. of 50,000 for alpha-1-antitrypsin/M.W. of 23,000 for trypsin).

Conclusions

1. Dialysis of Cohn IV-I solutions to remove alcohol prior to salting out is not necessary.

2. DTT is highly effective in providing a medium for protein precipitation without adversely affecting the alpha-1-antitrypsin recovery in the supernatant.

3. Protein concentrations in the range of 20—50 mg/ml are selectively salted out similarly and quite effectively.

Example 2

To maximize the recovery of antitrypsin, Cohn IV-I was activated as described in Example 1 above without dialysis. The activated solution contained ~27.75 mg/ml of protein as determined by the Lowry method. The following DTT concentrations were used: 0, 0.2M, 0.03M, 0.05M, 0.01M, 0.20M. For each DTT concentration, separate incubation was performed for 3, 8 and 24 hours. After reduction, each 10 ml aliquot was precipitated with 10 ml of saturated $(NH_4)_2SO_4$ to a final concentration of 50% at 5°C and incubated for 24 hours, then centrifuged, washed, recentrifuged. The supernatants were dialysed against 1×4 liter 0.01M phosphate buffer, pH 7.6 containing 0.01M cysteine to reform the disulfide bridge with alpha-1-antitrypsin. A final dialysis against 3×4 liter 0.01M phosphate buffer was used to remove excess cysteine.

Conclusions

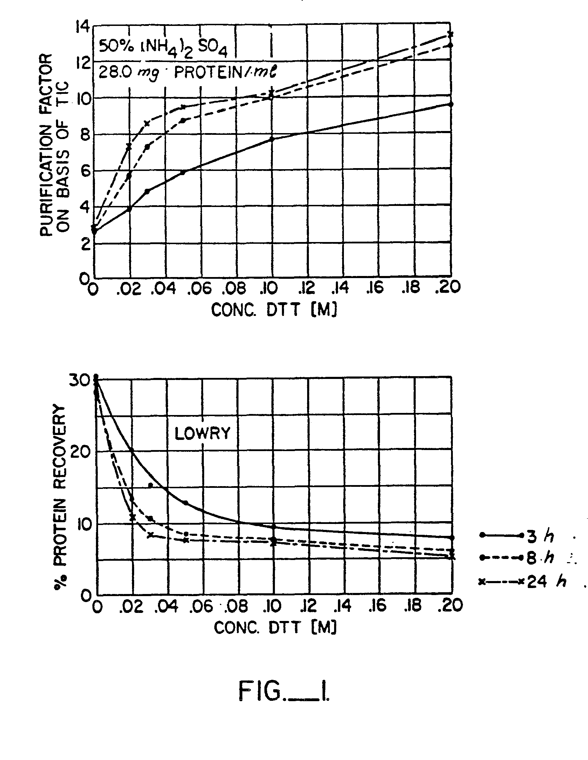

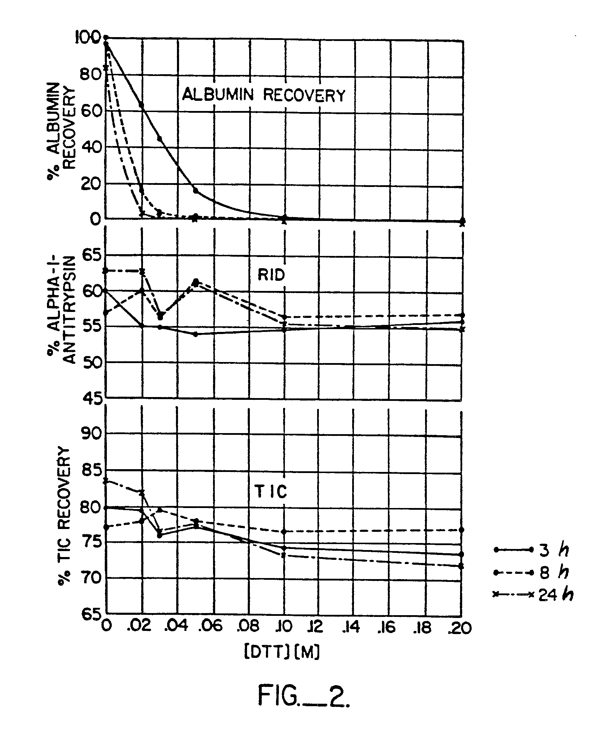

The results are seen in Figs. 1 and 2 and indicate:

1. Reduction with dithiothreitol (DTT), followed by precipitation with $(NH_4)_2SO_4$ (50%) is highly effective in removing contaminating proteins from alpha-1-antitrypsin containing protein solutions of Cohn IV-I.

2. The effectiveness of the method is dependent on the concentration of reductant and on the time of reduction. 0.05M DTT treatment for 8 hours is highly effective, giving a purification factor >8.0 (as compared to 3.0 for the control). Higher levels of DTT are somewhat better, but at an increased cost factor and with only modest improvement.

3. Albumin is completely removed by this treatment.

4. The recovery of alpha-1-antitrypsin activity is 75—80% as determined by TIC, and this closely parallels the recovery found in the absence of DTT.

5. The recovery of immunological activity (radial immunodiffusion) is ~55—60%. (Note: This is the result of some aggregated alpha-1-antitrypsin in the original Cohn IV-I solution, as will be demonstrated in Example 3.

Example 3

To determine whether any antitrypsin aggregate is present in Cohn IV-I, an aliquot of 0.6 ml of Cohn IV-I solution, containing 25.3 mg/ml of protein and 1.7 mg/ml of alpha-1-antitrypsin (determined by TIC), was applied onto a Sephadex® G-150 column (1.5×91 cm) which had been equilibrated with 0.10M $NH_4HCO_3$.

Based on a TIC assay on the eluants, fractions 18—29 had only traces of inhibitory activity, and these were pooled (peak A). Fractions 30—40 containing most of the activity were combined (peak B).

TABLE II

|  | Total protein (Lowry) mg | Total alpha-1-antitrypsin (RID) mg | Total alpha-1-antitrypsin (TIC-corr.) mg | RID/TIC |
|---|---|---|---|---|
| Peak A | 8.4 | 0.258 | 0.137 | 1.90 |
| Peak B | 3.6 | 0.554 | 0.582 | 0.95 |

RID(A) and+RID(B)=100

$$\% \text{ aggregate} = \frac{0.258}{0.258+.554} \times 100 = 32\%$$

Conclusions

It appears that Cohn IV-I contains approximately 32% aggregate. This is only an approximation for two reasons: 1) The radial immuno-diffusion assay (RID) will not distinguish between monomeric and polymeric alpha-1-antitrypsin. Polymeric alpha-1-antitrypsin will diffuse less in the agar gel and its concentration will be underestimated. 2) Since the column was not optimal, some monomer could be eluting in peak A, and this would tend to overestimate the alpha-1-antitrypsin polymer. Alternatively, the limited trypsin inhibiting activity seen in peak A may be the result of other higher MW inhibitors known to be present in Cohn IV-I, i.e., alpha-2-macroglobulin, inter-alpha trypsin inhibitor.

Example 4

To test the effect of reduction and salting out on the antitrypsin aggregate, supernatant from reduced-salted out Cohn IV-I was dialysed against 0.1M NH₄HCO₃ and a one ml aliquot containing 19.1 mg of protein and 9.6 mg alpha-1-antitrypsin was applied into the Sephadex® G-150 column (1.5×91 cm) equilibrated in the same buffer.

The fractions were pooled and labeled A (aggregate peak) and B, and analyzed by various assays as indicated by the summary below.

TABLE III

|  | Total alpha-1-antitrypsin RID mg | Total alpha-1-antitrypsin TIC (corrected) mg |
|---|---|---|
| A | 0 | 0 |
| B | 4.42 | 5.5 |

In conclusion, it seems clear that treatment of Cohn IV-I with DTT followed by $(NH_4)_2SO_4$ salting out effectively removes all aggregated alpha-1-antitrypsin.

Example 5

To further determine whether the time of reduction and/or the molarity of reducing agent could be decreased in the salting out scheme, the process was run at 37°C.

Activated Cohn IV-I was divided into 9.5 ml aliquots containing 31.9 mg/ml protein and treated at 37°C for 2, 4 or 8 hours with various concentrations of DTT, or cysteine. Control solutions containing no reductant were also included. All aliquots were then treated to a final concentration of 50% $(NH_4)_2SO_4$, incubated for 24 hours at 5°C, centrifuged at 10,000 rpm and the precipitates washed with cold 50% $(NH_4)_2SO_4$. The combined supernatants were dialysed against 1×4 l of 0.01M phosphate pH 7.6 containing 0.01 M cysteine followed by 3×4 l 0.01M phosphate, pH 7.6 without cysteine. The assays performed were TIC, Lowry and albumin radial immunodiffusion. A summary of the results is given in Table IV.

TABLE IV

| | % Alpha-1-anti-trypsin recovery (by TIC) | % Protein recovery (Lowry) | % Albumin recovery (RID) | Purification factor |
|---|---|---|---|---|
| **2 Hours** | | | | |
| Control | 88.3 | 33.0 | 99.5 | 3.0 |
| 0.01M DTT | 78.6 | 17.0 | 2.0 | 5.3 |
| 0.05M DTT | 78.6 | 11.3 | 0 | 7.3 |
| 0.1M DTT | 70.9 | 10.3 | 0 | 7.5 |
| 0.1M cysteine | 83.5 | 24.0 | 95.0 | 3.3 |
| 1.0M cysteine | 73.3 | 16.0 | 21.0 | 5.0 |
| **4 Hours** | | | | |
| Control | 89.0 | 31.5 | 99.5 | 3.0 |
| 0.01M DTT | 74.2 | 13.0 | 7.7 | 6.3 |
| 0.05M DTT | 68.0 | 10.8 | 0 | 6.8 |
| 0.1M DTT | 74.2 | 9.6 | 0 | 8.5 |
| 0.1M cysteine | 84.8 | 25.8 | 89.3 | 3.5 |
| 1.0M cysteine | 74.2 | 12.9 | 1.5 | 6.3 |
| **8 Hours** | | | | |
| Control | 94.8 | 32.0 | 100.0 | 3.3 |
| 0.01M DTT | 76.8 | 13.0 | 1.0 | 6.3 |
| 0.05M DTT | 80.0 | 11.3 | 0 | 7.5 |
| 0.1M DTT | 73.8 | 9.4 | 0 | 8.5 |
| 0.1M cysteine | 91.5 | 24.7 | 99.0 | 4.0 |
| 1.0M cysteine | 79.8 | 14.9 | 0 | 5.8 |

Conclusions

The purification factors obtained with DTT at 37°C for 4—8 hours were similar to those at 25°C for 24 hours. Cysteine, even at 1M, is not as effective as 0.05M DTT.

Example 6

To test the effect of pH on salting out after reduction, Cohn IV-I was activated without the removal of ethanol. For this experiment, salting out was carried out at pH 6.0 and at pH 8.5.

80 ml of Cohn IV-I in 0.1M phosphate pH 8.5 (15 mg/ml protein by Lowry) was divided into two 40 ml samples (A and B). Sample A was treated with 0.1M DTT for 24 hours at room temperature. Sample B was kept as a control (no DTT). Following reduction, sample A was divided into two equal aliquots $A_1$ and $A_2$. $A_1$ was titrated under stirring with 1M HCl to pH 6.0 (final volume 22.8 ml). $A_2$ was diluted with pH 8.5 in order to maintain the same protein concentration as $A_1$. The control sample B was treated similarly. From each aliquot ($A_1$, $A_2$, $B_1$, $B_2$) 20 ml were removed and treated with $(NH_4)_2SO_4$ at 5°C to a final concentration of 50% salt. After 24 hours of incubation, the solid ppts were centrifuged at 10,000 rpm, washed and dialyzed against 1×4 l 0.01M phosphate, pH 7.6 containing 0.01M cysteine and 3×4 l 0.01M phosphate pH 7.6.

TABLE V

| | DTT | pH for salting out | Alpha-1-antitrypsin recovery % | Protein recovery (Lowry) % | Purification factor | Albumin recovery (RID) % |
|---|---|---|---|---|---|---|
| A₁ | + | 6.0 | 64 | 8 | 8 | 0 |
| A₂ | + | 8.5 | 85 | 10 | 8 | 0 |
| B₁ | − | 6.0 | 70 | 27 | 2.5 | 90 |
| B₂ | − | 8.5 | 83 | 30 | 2.8 | 90 |

Conclusions

Adjustment of the pH to 6 prior to salting out leads to somewhat greater precipitation of overall protein; however the precipitation of alpha-1-antitrypsin is also increased. The net effect is that the purification factor is approximately the same at pH 6 and 8.5. Since the loss of alpha-1-antitrypsin is greater at pH 6, salting out at pH 8.5 seems preferable.

Example 7

To determine the purification factor achievable by reduction and salting out in plasma, citrated plasma was diluted 2, 4 or 6-fold, and each solution divided into two equal parts. One part was reduced with 0.1M DTT for 24 hours at 25°C and then salted out to 50% $(NH_4)_2SO_4$. The second part of each diluted plasma was incubated and salted out, but not reduced. The reduced aliquots A, B, C had insoluble protein which were readily removed by centrifugation at 10,000 rpm (5°C) for 20 minutes. The supernatants and washes were combined, treated to 50% $(NH_4)_2SO_4$ saturation, incubated for 24 hours at 5°C and centrifuged as before. The controls were also similarly treated with $(NH_4)_2SO_4$, etc. Each solution was dialysed against 1×4 l phosphate pH 7.6 containing 0.01M cysteine and then phosphate alone.

TABLE VI

| | 0.1M DTT | Dilution | Protein (Lowry) initial | final | % Recovery (Protein) | (TIC) | Purification factor |
|---|---|---|---|---|---|---|---|
| A | + | 2-fold | 501.0 | 46.2 | 9 | 61 | 6.66 |
| B | + | 4-fold | 250.5 | 17.9 | 7 | 68 | 9.60 |
| C | + | 6-fold | 167.0 | 10.6 | 6.3 | 72 | 11.40 |
| D | − | 2-fold | 501.0 | 307.8 | 61.0 | 65 | 1.07 |
| E | − | 4-fold | 250.5 | 161.0 | 64.0 | 78 | 1.22 |
| F | − | 6-fold | 167 | 121.6 | 73.0 | 81 | 1.12 |

Conclusions

The reduction-salting out scheme gives a greatly enhanced purification factor in plasma. Dilute plasma solutions give better results. Albumin is essentially completely precipitated by the reduction protocol as was shown by radial-immunodiffusion.

Example 8

To illustrate separation of alpha-1-antitrypsin from the supernatant. Cohn IV-I was activated, reduced and salted out essentially as described in the previous examples. The solution (770 ml) obtained contained ~2.77 mg/ml of protein, of which ~50% was alpha-1-antitrypsin. A 20 ml DEAE-cellulose column was packed and equilibrated with 0.01M phosphate, pH 7.6. TIC was used to monitor the eluant fractions for antitrypsin activity. 664 ml of solution were loaded corresponding to a maximum column capacity of 1.84 gm of total protein or 92 mg of protein/ml of resin. The resin was then washed with starting buffer to remove unbound protein, and then eluted with 400 ml gradient consisting of 0.01M phosphate, pH 7.6→0.01M phosphate+0.07M NaCl, pH 6.0.

After monitoring OD and TIC, active fractions (60—115) were pooled. Approximately 597 mg (~32%) of the protein did not bind to the resin.

A summary of the results is included below.

TABLE VII

| | Protein (Lowry) mg | Alpha-1- antitrypsin corr. (TIC) | Specific activity | Alpha-1- antitrypsin yield % | Purifi- cation factor |
|---|---|---|---|---|---|
| Cohn IV-I | 26,500 | 1,709 | 0.064 | — | — |
| DTT+$(NH_4)_2SO_4$ | 2,132 | 1,115 | 0.523 | 65 | 8.15 |
| DEAE | 551 | 516 | 0.936 | 32 | 14.6 |

Conclusions

The product obtained was quite pure (Spec. Activity 0.94 mg antitrypsin/mg protein. This example also illustrates that an overloaded column can be effectively utilized.

Example 9

The activation, reduction and salting out of Cohn IV-I was performed as described in the previous examples.

A 23 ml column of DEAE-cellulose was equilibrated with 0.01M phosphate buffer, pH 7.6, and 430 ml of protein solution, corresponding to 25.7 mg of protein/ml of resin was passed through. The unbound proteins were washed out with starting buffer and column eluted with 500 ml gradient consisting of 0.01M phosphate, pH=7.6→0.01M phosphate+0.1M NaCl, pH=6.0. After obtaining a protein elution pattern and checking proteins for activity, tubes 175—210 were pooled.

A summary of the results is found in Table VIII.

TABLE VIII

| | Protein (Lowry) mg | Alpha-1- antitrypsin mg | Specific activity | Alpha-1- antitrypsin yield % | Purifi- cation factor |
|---|---|---|---|---|---|
| Cohn IV-I | 6507 | 421.5 | 0.07 | — | — |
| DTT+$(NH_4)_2SO_4$ | 591.2 | 254.3 | 0.43 | 60 | 6.15 |
| DEAE | 198.8 | 212.7 | 1.07 | 50.5 | 15.3 |

Conclusions

The non-overloaded DEAE procedure gives an excellent product of high purity and with 84% recovery based on the amount of alpha-1-antitrypsin loaded on the column. It is evident that significant quantities of antichymotrypsin bind tightly to this column and can be separated if desired.

Example 10

Activation, reduction and salting out of Cohn IV-I solution was performed as described in previous examples. 365 ml of DTT/$(NH_4)_2SO_4$ treated protein solution in 0.01M phosphate, 0.01M cysteine pH 7.6 containing 16.3 mg of protein/ml and 5.9 mg alpha-1-antitrypsin/ml was concentrated by ultra-filtration to ~250 ml. The solution was diafiltered against 1980 ml of 0.05M $NH_4HCO_3$ using a 100,000 M.W. cutoff hollow fiber. Both the filtrate and the solution remaining within the chamber were examined for alpha-1-antitrypsin content and purity, and compared to alpha-1-antitrypsin obtained from DEAE-cellulose.

TABLE IX

| | Protein mg | Alpha-1-anti- trypsin mg (TIC) | Recovery % | Specific TIC mg/mg | Purifi- cation factor |
|---|---|---|---|---|---|
| DTT $(NH_4)_2SO_4$ | 5,949 | 2,156 | | 0.362 | |
| Amicon ultrafiltration (Filtrate) | 2,831 | 1,881 | 87 | 0.66 | 1.8 |
| (Retained solution) | 2,825 | 304 | 14 | 0.109 | |
| DEAE-cellulose | | | 91 | 0.894 | 2.4 |

Using Amicon ultrafiltration at 100,000 M.W. cutoff 87% of alpha-1-antitrypsin passed through the membrane and a 1.8-fold purification factor was obtained. An SDS-polyacrylamide gel of purified protein showed one major impurity of higher M.W., probably alpha-1-antitrypsin aggregate. On the other hand the material purified by DEAE-cellulose appeared homogenous. Purity of the product was also examined by immunoelectrophoresis using antibodies to different plasma proteins. Inhibitor purified by DEAE-cellulose showed contamination with antithrombin III and alpha-1-lipoprotein; the Amicon purified alpha-1-antitrypsin in addition to these impurities also contains alpha-1-antichymotrypsin, alpha-1-acid glycoprotein, and $C_1S$ inactivator.

Conclusions

The DEAE-cellulose purification step appears superior to the ultrafiltration preparation by purity (89% vs. 70—75%) and recovery (91 vs. 87%). On the other hand, ultrafiltration purification is extremely rapid, simple, inexpensive and can be scaled up easily.

Example 11

In a publication by Laurell, et al, (*Eur. J. Biochem.*, 57:107—113, 1975) the authors treat plasma with 30 mM beta-mercaptoethanol to prepare alpha-1-antitrypsin for thiol-disulfide exchange. They follow this step with ammonium sulfate fractionation to partially purify the alpha-1-antitrypsin prior to the thiol chromatography step. This Example compares the effectiveness of beta-mercaptoethanol versus DTT as reductant using the conditions described by Laurell, et al., and those used in this application. Laurell, et al., incubated with 30 mM beta-mercaptoethanol followed by fractionation with 50% saturated $(NH_4)_2SO_4$ at 25°C; the present method involves incubation with 100 mM DTT for 24 hours followed by 50% saturated $(NH_4)_2SO_4$ at 4°C.

The following matrix experiment was conducted:

4.0 ml of reductant in aqueous solution were added to 2.0 ml aliquots of plasma to yield the final reductant concentrations (30 or 100 mM). These treated plasma samples were incubated for one hour or 24 hours at 25°C, and then 6.0 ml of 100% saturated $(NH_4)_2SO_4$ solutions were added at 25°C or 4°C, respectively. After sixteen hours, the samples were centrifuged at 3,000×g for twenty minutes, washed with 50% saturated $(NH_4)_2SO_4$ and recentrifuged. Supernatants were dialyzed against 10 mM sodium phosphate (pH 7.6) buffer containing 10 mM cysteine followed by 10 mM phosphate (pH 7.6) alone. Lowry and TIC assays were done on the dialyzed supernatants and on a plasma control. The results are presented in Table X.

TABLE X

Comparison of beta-mercaptoethanol (BME) and DTT as reductant in the purification of alpha-1-antitrypsin from plasma

| Sample | mg Alpha-1-antitrypsin | mg protein | Specific activity | Purification factor |
|---|---|---|---|---|
| Plasma control | 2.5 | 131.2 | 0.019 | 1 |
| One-hour reduction | | | | |
| No reductant | 1.95 | 66.8 | 0.029 | 1.5 |
| 30 mM BME | 2.17 | 72.5 | 0.030 | 1.6 |
| 30 mM DTT | 1.96 | 18.0 | 0.109 | 5.7 |
| 100 mM BME | 1.86 | 71.1 | 0.026 | 1.4 |
| 100 mM DTT | 1.76 | 8.1 | 0.217 | 11.4 |
| 24-Hour reduction | | | | |
| No reductant | 2.08 | 72.0 | 0.029 | 1.5 |
| 30 mM BME | 1.96 | 71.3 | 0.027 | 1.4 |
| 30 mM DTT | 0.98 | 4.55 | 0.215 | 11.3 |
| 100 mM MBE | 1.88 | 53.0 | 0.035 | 1.8 |
| 100 mM DTT | 1.07 | 5.28 | 0.203 | 10.7 |

Conclusions

The results of Table X imply the following conclusions:

1. With a one-hour incubation with reductant and salting out at 25°C:

a. DTT plus $(NH_4)_2SO_4$ greatly enhances the purification of alpha-1-antitrypsin from plasma over the purification yielded by 50% saturated $(NH_4)_2SO_4$ alone. 100 mM DTT is more effective than 30 mM DTT during the short incubation period.

b. Beta-mercaptoethanol in conjunction with 50% saturated $(NH_4)_2SO_4$ yields no improvement in purification of alpha-1-antitrypsin over $(NH_4)_2SO_4$ alone. Neither 100 mM beta-mercaptoethanol nor 30 mM beta-mercaptoethanol is effective.

c. The conditions of Laurell, et al., (30 mM beta-mercaptoethanol, 1 hour incubation, 50% saturated $(NH_4)_2SO_4$, at room temperature [~22°C]) yield no improvement over $(NH_4)_2SO_4$ alone in the purification of alpha-1-antitrypsin. The use of beta-mercaptoethanol in their procedure is to reduce alpha-1-antitrypsin for subsequent thiol exchange chromatography, not to enhance the purification of alpha-1-antitrypsin by destabilizing contaminant proteins.

2. With 24 hours of incubation with reductant and salting out at 4°C:

a. DTT plus 50% saturated $(NH_4)_2SO_4$ greatly enhances the purification of alpha-1-antitrypsin from plasma over the purification yielded by $(NH_4)_2SO_4$ alone. 30 mM DTT is as effective as 100 mM DTT at this incubation time.

b. Beta-mercaptoethanol plus 50% saturated $(NH_4)_2SO_4$, even at 1 hour of reduction, yields substantially no improvement in purification of alpha-1-antitrypsin over $(NH_4)_2SO_4$ alone, with a small improvement apparently commencing at 24 hours and 100 mM.

c. The yield of alpha-1-antitrypsin in the DTT-treated samples of three-fold diluted plasma incubated with reductant for 24 hours is ~40%, due to the formation of a firm clot. This effect of DTT on alpha-1-antitrypsin yield is not noted in samples incubated for one hour nor, as shown in previous work, is it noted in six-fold diluted plasma.

Example 12

A second matrix experiment was conducted to determine whether DTT alone is sufficient to effect a purification of alpha-1-antitrypsin from plasma comparable to the purification achieved by the reductive-salting out scheme. Pierce and Eradio (*J. Lab. Clin. Med.* **94**:826—831, 1979) used DTT to remove albumin from plasma samples prior to phenotyping alpha-1-antitrypsin by isoelectric focusing. They found that 30 mM DTT for one hour at 37°C precipitated albumin in a firm clot. This experiment shows that the coupling of DTT and $(NH_4)_2SO_4$ exhibits a greater alpha-1-antitrypsin purification than DTT alone, using 30 and 100 mM concentrations of reductant.

0.2 ml of reductant in aqueous solution were added to 2.0 ml aliquots of plasma to yield the DTT concentrations given above. The reductant-treated plasma samples were incubated from one hour at 37°C and then either were centrifuged at 3000×g or were fractionated with 50% saturated $(NH_4)_2SO_4$ and then centrifuged. The supernatants were dialyzed versus 10 mM sodium phosphate (pH 7.6)+10 mM cysteine buffer followed by 10 mM phosphate (pH 7.6). The samples, plus a plasma control, were assayed by Lowry and TIC with the results compiled in Table XI.

TABLE XI

Comparison of DTT alone and DTT plus 50% saturated $(NH_4)_2SO_4$ in the purification of alpha-1-antitrypsin from plasma

| Sample | mg Alpha-1-antitrypsin | mg Protein | Specific activity | Purification factor |
|---|---|---|---|---|
| No $(NH_4)_2SO_4$ | | | | |
| no DTT | 1.62 | 88.9 | 0.018 | 1.0 |
| 30 mM DTT | 0.60 | 13.2 | 0.045 | 2.5 |
| 100 mM DTT | 0.64 | 14.9 | 0.043 | 2.4 |
| +$(NH_4)_2SO_4$ | | | | |
| No DTT | 1.55 | 44.6 | 0.035 | 1.9 |
| 30 mM DTT | 0.61 | 3.26 | 0.188 | 10.4 |
| 100 mM DTT | 0.49 | 3.17 | 0.155 | 8.6 |

Conclusions

1. DTT alone yields a slight purification of alpha-1-antitrypsin from undiluted plasma.

2. DTT plus 50% saturated $(NH_4)_2SO_4$ improves the purification of alpha-1-antitrypsin ~four-fold over the purification shown by DTT alone.

3. The conditions of Pierce and Eradio (30 mM DTT, one hour at 37°C) do not demonstrate the dramatic enhancement of alpha-1-antitrypsin purification shown by the combination of DTT reduction plus fractionation by 50% saturated $(NH_4)_2SO_4$.

4. The low yields of alpha-1-antitrypsin (30—40%) from undiluted plasma incubated with DTT results from clot formation. This effect of DTT, as shown previously, is not seen in six-fold diluted plasma.

Example 13

To test whether Aerosil®, a fumed silica product, can improve the purification of alpha-1-antitrypsin in the reduction salting out method, and to determine whether the product retains activity against elastase (in addition to trypsin) the following experiment was performed.

Activation of alpha-1-antitrypsin in Cohn IV-I was accomplished by dissolving 50 gm of Cohn IV-I paste in 0.5 l of 0.1M Tris-HCl pH 8.5) and allowing the solution to stir overnight at 25°C. Dithiothreitol at a concentration of 25 mM was added to activated Cohn IV-I in the presence of 2.5% Aerosil 380® (Degussa, Inc., Teterboro, N.J.) at 25°C with stirring for 24 hours. Aerosil 380® is a fumed-silica material reported to be effective in removing alpha- and beta-lipoproteins and other beta-globulins from aqueous solutions. The dithiothreitol treated solution was salted out in 50% saturated ammonium sulfate (4°C) and, after equilibration overnight, was centrifuged at 3000×g for one hour. The precipitate was washed with 50% saturated $(NH_4)_2SO_4$ and recentrifuged. The combined supernatants were dialyzed against 10 mM sodium phosphate (pH 7.6), containing 10 mM cysteine, followed by dialysis against 10 mM sodium phosphate (pH 7.6).

TABLE XII

| | Protein (Lowry) mg | Alpha-1-antitrypsin mg | | Alpha-1-anti-trypsin yield (%) | | Purification factor | |
|---|---|---|---|---|---|---|---|
| | | TIC | EIC* | TIC | EIC | TIC | EIC |
| Cohn IV-I | 15,200 | 735 | 634 | — | — | — | — |
| DTT+$(NH_4)_2SO_4$+Aerosil® | 764 | 512 | 447 | 69.6 | 70.5 | 13.7 | 13.9 |

*The elastase inhibitory capacity (EIC) was determined against porcine pancreatic elastase (Calbiochem-Behring) using p-nitrophenyl N - tert - butyloxycarbonyl - L - alaninate as substrate. Visser, L., and Blout, E. R. (1972) *Biochim. Biophys. Acta,* 268; 257—260.

Conclusions

1. The Aerosil® method does appear to offer advantages in removing contaminant proteins to give a higher purification factor (~13.7, 13.9 versus 8.15 in Table VII). The recovery of alpha-1-antitrypsin was as good as experiments performed in the absence of Aerosil®.

2. The product had an elastase inhibitory capacity which paralleled the trypsin inhibitory capacity.

**Claims**

1. A method for isolating alpha-1-antitrypsin from a mixture of blood plasma proteins in solution comprising:
(a) breaking sufficient disulfide bonds in said proteins to destabilize them,
(b) precipitating the destabilized proteins,
(c) and recovering non-precipitated alpha-1-antitrypsin from said solution.

2. A method for isolating alpha-1-antitrypsin in accordance with claim 1 wherein said disulfide bonds are broken by treating the solution with an effective concentration of a reducing agent having a reducing strength sufficient to reduce disulfide bonds but insufficient to reduce other protein bonds or groups so as to fragment the protein.

3. A method for isolating alpha-1-antitrypsin in accordance with claim 2 wherein said reducing agent is selected from monothiols and dithiols.

4. A method for isolating alpha-1-antitrypsin in accordance with claim 3 wherein said monothiol is present in a concentration of 0.1—1.5M and said dithiol is present in a concentration of 0.01—0.1M.

5. A method for isolating alpha-1-antitrypsin in accordance with claim 2 wherein said reducing agent is selected from dithiothreitol and dithioerythritol.

6. A method for isolating alpha-1-antitrypsin in accordance with claim 5 wherein said reducing agent is added to the solution in a concentration of 0.03—0.10M.

7. A method for isolating alpha-1-antitrypsin in accordance with claim 6 wherein the reduction reaction is executed at 25—37°C for 2—24 hours.

13

8. A method for isolating alpha-1-antitrypsin in accordance with any one of claims 1—7 wherein the destabilized proteins are precipitated by salting them out.

9. A method for isolating alpha-1-antitrypsin in accordance with any one of claims 1—8 wherein said mixture of blood plasma proteins is a human plasma Cohn fraction IV-I.

## Patentansprüche

1. Verfahren zur Isolierung von Alpha-1-antitrypsin aus einem Gemisch aus Blutplasmaproteinen in Lösung, welches (folgende Stufen) umfaßt:

a) Aufbrechen einer ausreichenden Menge von Disulfid-Bindungen in den Proteinen, um diese zu stabilisieren,

b) Ausfällen der destabilisierten Proteine,

c) und Gewinnung des nicht-ausgefällten Alpha-1-antitrypsins aus der Lösung.

2. Verfahren zur Isolierung von Alpha-1-antitrypsin nach Anspruch 1, worin die Disulfidbindungen dadurch aufgebrochen werden, daß die Lösung mit einer wirksamen Konzentration eines Reduktionsmittels behandelt wird, dessen Reduktionsvermögen ausreicht, um die Disulfidbindungen zu reduzieren, jedoch nicht ausreicht, um andere Proteinbindungen oder -gruppen unter Bildung von Proteinfragmenten zu reduzieren.

3. Verfahren zur Isolierung von Alpha-1-antitrypsin nach Anspruch 2, worin das Reduktionsmittel aus Monothiolen und Dithiolen ausgewählt ist.

4. Verfahren zur Isolierung von Alpha-1-antitrypsin nach Anspruch 3, worin das Monothiol in einer Konzentration von 0,1 bis 1,5 M und das Dithiol in einer Konzentration von 0,01 bis 0,01 M vorhanden ist.

5. Verfahren zur Isolierung von Alpha-1-antitrypsin nach Anspruch 2, worin das Reduktionsmittel aus der Gruppe Dithiothrit und Dithioerythrit ausgewählt ist.

6. Verfahren zur Isolierung von Alpha-1-antitrypsin nach Anspruch 5, worin das Reduktionsmittel der Lösung in einer Konzentration von 0,03 bis 0,10 m zugesetzt wird.

7. Verfahren zur Isolierung von Alpha-1-antitrypsin nach Anspruch 6, worin die Reduktionsreaktion bei 25 bis 37°C über 2 bis 24 Std. durchgeführt wird.

8. Verfahren zur Isolierung von Alpha-1-antitrypsin nach einem der Ansprüche 1 bis 7, worin die destabilisierten Proteine durch Aussalzen ausgefällt werden.

9. Verfahren zur Isolierung von Alpha-1-antitrypsin nach einem der Ansprüche 1 bis 8, worin das Gemisch aus Blutplasmaproteinen eine menschliche Cohn-Plasma-Fraktion IV-I darstellt.

## Revendications

1. Procédé pour isoler l'apha-1-antitrypsine d'un mélange de protéines du plasma sanguin en solution, comprenant:

(a) la rupture de suffisamment de liaisons disulfures dans lesdites protéines pour destabiliser celles-ci,

(b) la précipitation des protéines déstabilisées,

(c) et la récupération, à partir de ladite solution, de l'apha-1-antitrypsine non précipitée.

2. Procédé pour isoler l'alpha-1-antitrypsine selon la revendication 1, selon lequel on rompt les liaisons disulfures en traitant la solution par une concentration efficace d'un agent réducteur ayant un pouvoir réducteur suffisant pour réduire des liaisons disulfures mais ne suffisant pas pour réduire d'autres liaisons ou groupes de protéines de manière à fragmenter la proténe.

3. Procédé pour isoler l'alpha-1-antitrypsine selon la revendication 2, selon lequel ledit agent réducteur est choisi parmi des monothiols et des dithiols.

4. Procédé pour isoler l'alpha-1-antitrypsine selon la revendication 3, dans lequel ledit monothiol est présent en une concentration de 0,1 à 1,5M et ledit dithiol est présent en une concentration de 0,01 à 0,1M.

5. Procédé pour isoler l'alpha-1-antitrypsine selon la revendication 2, dans lequel ledit agent réducteur est choisi parmi le dithiothreitol et le dithioérythritol.

6. Procédé pour isoler l'alpha-1-antitrypsine selon la revendication 5, dans lequel ledit agent réducteur est ajouté à la solution en une concentration de 0,03 à 0,10M.

7. Procédé pour isoler l'alpha-1-antitrypsine selon la revendication 6, selon lequel on effectue la réaction de réduction à 25—37°C durant 2 à 24 heures.

8. Procédé pour isoler l'alpha-1-antitrypsine selon l'une quelconque des revendications 1 à 7, dans lequel on précipite par relargage les protéines déstabilisées.

9. Procédé pour isoler l'alpha-1-antitrypsine selon l'une quelconque des revendications 1 à 8, dans lequel ledit mélange de protéines du plasma humain est une fraction IV-I de Cohn de plasma humain.

FIG.__I.

FIG.__2.